## Europäisches Patentamt
## European Patent Office
## Office européen des brevets

(19)

(11) Veröffentlichungsnummer: **0 238 961 B1**

(12)

# EUROPÄISCHE PATENTSCHRIFT

(45) Veröffentlichungstag der Patentschrift: **11.11.92**

(51) Int. Cl.5: **C07C 213/00**, C07C 215/12, C07C 249/02

(21) Anmeldenummer: **87103748.7**

(22) Anmeldetag: **14.03.87**

(54) **Verfahren zur Herstellung von Hydroxyaminen.**

(30) Priorität: **25.03.86 DE 3609978**

(43) Veröffentlichungstag der Anmeldung:
**30.09.87 Patentblatt 87/40**

(45) Bekanntmachung des Hinweises auf die
Patenterteilung:
**11.11.92 Patentblatt 92/46**

(84) Benannte Vertragsstaaten:
**AT BE CH DE FR GB IT LI NL SE**

(56) Entgegenhaltungen:
DE-A- 2 829 916
US-A- 4 540 821

(73) Patentinhaber: **MERCK PATENT GESELL-
SCHAFT MIT BESCHRÄNKTER HAFTUNG
Frankfurter Strasse 250 Postfach 4119
W-6100 Darmstadt(DE)**

(72) Erfinder: **Felder, Ernst, Prof.
Via Ceresio 49
CH-6826 Riva San Vitale(CH)**
Erfinder: **Römer, Michael, Dr.
Niederwiesenring 129a
W-6054 Rodgau 2(DE)**
Erfinder: **Bardonner, Hans
Erbacherstrasse 12
W-6123 Bad König(DE)**
Erfinder: **Härtner, Hartmut, Dr.
Mathildenweg 9
W-6109 Mühltal 4(DE)**
Erfinder: **Fruhstorfer, Wolfgang, Dr.
Heideweg 4a
W-6109 Mühltal-Traisa(DE)**

## Beschreibung

Die Erfindung betrifft ein neues Verfahren zur Herstellung von Hydroxyaminen der Formel I,

$$R^1\text{-CH(OH)-CH(NHR}^3)\text{-R}^2 \quad I$$

worin $R^1$ und $R^2$ jeweils unabhängig voneinander H, $CH_2OH$ und $R^3$ H, $C_1$-$C_4$ Alkyl, $C_1$-$C_4$ Hydroxyalkyl bedeuten.

Hydroxyamine der Formel I stellen wertvolle Zwischenprodukte zur Herstellung von Arzneimittelwirkstoffen dar.

So findet beispielsweise 2-Amino-1,3-propandiol (Serinol) Verwendung zur Herstellung des Röntgenkontrastmittels Jopamidol [N,N'-Bis-[2-hydroxy-1-(hydroxymethyl)-ethyl] -2,4,6-triiod-5-lactamido-isophthalamid].

Eine Vielzahl von Herstellungsverfahren für Hydroxyamine der Formel I ist bekannt. So sind diese Verbindungen darstellbar durch Reduktion entsprechender Nitro- oder Oximinoderivate von Aminosäuren oder deren Ester oder von Oximinodicarbonsäureestern. Auch die Reaktion von Formaldehyd mit geeigneten Monohydroxyaminen in Gegenwart bestimmter Mikroorganismen oder durch Sulfatierung von Glycerin und anschließender Ammonolyse sind bekannt.

Neben Hydrierverfahren ist in der EP-O 025 083 eine Herstellung ausgehend von dem cancerogenen Epichlorhydrin offenbart.

Die beschriebenen Syntheseverfahren zeigen jedoch eine Reihe von Nachteilen. Entweder sind die Ausbeuten unbefriedigend, oder die Ausgangsstoffe sind teuer oder nur auf umständlichem Wege zugänglich oder beeinträchtigen stark die Gesundheit, oder es fallen Nebenprodukte an, beispielsweise Salze, die schwierig oder nur mit großem Aufwand entfernbar sind.

In der DE-28 29 916 wird ein Verfahren zur Herstellung von 2-Amino-1,3-propandiol durch Hydrierung von Dihydroxyaceton in Gegenwart von Ammoniak und eines Reduktionsmittels offenbart.

Dieses Verfahren liefert jedoch nur im kleinen Maßstab, bei Laborversuchen annehmbare Ausbeuten von etwa 60 % d. Th. Bei größeren, für eine industrielle Produktion geeigneten Reaktionsansätzen fallen die erzielbare Ausbeute und Qualität des Produkts aufgrund einer beträchtlichen Nebenproduktbildung stark ab. Darüberhinaus läßt sich der eingesetzte Katalysator infolge einer aktivitätsmindernden Beeinflussung durch die gebildeten Nebenprodukte meist nicht mehr wiederverwenden.

Der Erfindung lag die Aufgabe zugrunde, ein neues Verfahren zur Herstellung von I aufzufinden, das die Nachteile der bekannten Verfahren nicht oder nur in geringerem Maße besitzt. Diese Aufgabe wurde durch die Bereitstellung des neuen Verfahrens gelöst.

Es wurde gefunden, daß Hydroxyamine der Formel I vorteilhaft durch Umsetzung einer Oxoverbindung mit einem Amin zu einen Ketimin und dessen darauffolgende Behandlung mit einem Reduktionsmittel erhältlich sind. Die Ausbeuten bei diesem Verfahren sind ausgezeichnet und die Aufarbeitung gestaltet sich einfach, da keine oder nur sehr geringe Mengen an Nebenprodukten gebildet werden.

Gegenstand der Erfindung ist ein Verfahren zur Herstellung von Hydroxyaminen der Formel I,

$$R^1\text{-CH(OH)-CH(NHR}^3)\text{-R}^2 \quad I$$

worin $R^1$ und $R^2$ jeweils unabhängig voneinander H, $CH_2OH$ und $R^3$ H, $C_1$-$C_4$ Alkyl, $C_1$-$C_4$ Hydroxyalkyl bedeuten, dadurch gekennzeichnet, daß man eine Oxoverbindung der Formel II,

$$R^1\text{-CH(OH)-CO-R}^2 \quad II$$

worin $R^1$, $R^2$ die für Formel I angegebene Bedeutung besitzen, mit einem Amin $R^3$-$NH_2$, worin $R^3$ die für Formel I angegebene Bedeutung hat, zu einem Ketimin der Formel III umsetzt,

$$R^1\text{-CH(OH)-C(}=NR^3)\text{-R}^2 \quad III$$

worin $R^1$, $R^2$ und $R^3$ die für Formel I angegebene Bedeutung besitzen, und dieses anschließend mit einem Reduktionsmittel behandelt.

Überraschenderweise zeigt die erfindungsgemäße, zweistufige Verfahrensführung mit ihren längeren Reaktionszeiten im Vergleich zum Einstufenverfahren der DE-28 29 916 nahezu keine Nebenproduktbildung und damit erheblich bessere Ausbeuten und höhere Reinheiten der Hydroxyamine der Formel I.

Als Reduktionsmittel verwendet man vorzugsweise Wasserstoff in Gegenwart eines Metallkatalysators. Als Metalle sind Nickel, ferner Kobalt und Edelmetalle wie Platin, Rhodium, Palladium oder Ruthenium bevorzugt. Nickel- und Kobaltkatalysatoren werden zweckmäßig in Form von Raney-Metallen eingesetzt, die Edelmetallkatalysatoren in Form von Trägerkatalysatoren (z.B. Platin, Rhodium oder Palladium auf Kohle, Calciumkarbonat, Aluminiumoxid oder Strontiumcarbonat), in Form von Oxiden (z.B. Platin- oder Palladiumoxid) oder in feinverteilter Form (z.B. Platin-mohr). Die Menge des zu verwendenden Katalysators liegt etwa zwischen 1 und 100 Gewichtsprozent, bezogen auf den Ausgangsstoff der Formel II.

Die Amine der Formel $R^3$-$NH_2$ können in flüssiger oder gasförmiger Form oder gelöst in einem inerten Lösungsmittel in die Reaktion eingesetzt werden, zweckmäßig im Überschuß. Als Lösungsmittel sind Alkohole wie Methanol, Äthanol oder Isopropanol bevorzugt; ferner eignen sich z. B. Wasser, Äther wie Tetrahydrofuran oder Dioxan oder Gemische dieser Lösungsmittel. Man arbeitet zweckmäßig bei Drucken zwischen 1 und 300, vorzugsweise zwischen etwa 50 und 150 bar, und bei Temperaturen zwischen etwa 0 und 200°, vorzugsweise zwischen 40 und 90 °C. Ein Zusatz von Ammoniumsalzen, z. B. Ammoniumchlorid oder -acetat kann von Vorteil sein; zweckmäßig verwendet man 0,1 bis 1,5 Mol, bezogen auf 1 Mol Ausgangsstoff der Formel II.

Die Aufarbeitung ist bei dem erfindungsgemäßen Verfahren sehr einfach: man filtriert den Katalysator ab, dampft ein und reinigt die erhaltene Base I durch Destillation oder durch Kristallisation eines ihrer Salze. Der abfiltrierte Katalysator kann ohne Aktivitätsverlust erneut verwendet werden.

In der vorliegenden Erfindung steht somit ein sehr vorteilhaftes Verfahren zur einfachen und in hohen Ausbeuten verlaufenden Herstellung von Hydroxyaminen der Formel I, insbesondere zur Herstellung von 2-Amino-1,3-propandiol, aus Carbonylverbindungen der Formel II und Aminen der Formel $R^3$-$NH_2$ zur Verfügung.

Beispiel 1

In einer 1200 l-Apparatur werden 103,6 kg Dihydroxyaceton und 230 l Methanol vorgelegt und auf +10 °C abgekühlt. Bei max. 20 °C Innentemperatur werden 55 kg flüssiger Ammoniak eingetragen, wobei das Dihydroxyaceton in Lösung geht. Es wird 1 Stunde bei Raumtemperatur nachgerührt. Anschließend fügt man dem Reaktionsgemisch 100 kg feuchten Raney-Nickel-Katalysator zu und beaufschlagt die Apparatur mit einer Wasserstoffatmosphäre von 100 bar. Nach 40-minütigem Rühren bei 70 °C ist die Hydrierung beendet. Man läßt abkühlen und entfernt den Katalysator durch Filtration. Das Filtrat wird unter vermindertem Druck zum Rückstand eingedampft; es hinterbleiben 105 kg Roh-Serinol (Gehalt nach GC: 99 %; 99 % d. Th.).

Zur Reinigung wird mit Oxalsäuredihydrat in das Oxalat überführt; Ausbeute 136,5 kg (87,2 % d. Th.); F: 193-195°.

Beispiel 2

Man arbeitet analog Beispiel 1, verwendet als Ausgangsstoff jedoch ein entsprechendes Äquivalent Erythrulose. Man erhält ein Gemisch von 2-Desoxy-2-aminothreitol und 2-Desoxy-2-aminoerythritol in einer Gesamtausbeute von 89,4 % d.Th.

Beispiel 3

Aus Glycerinaldehyd und Ammoniak in Wasser wird analog Beispiel 1 das entsprechende Ketimin erhalten. Nach Zusatz von 10 Gew.-% bez. auf die Menge an Glycerinaldehyd eines 10 %igen Palladium/Aktivkohle-Katalysators wird bei 50° und 65 bar Wasserstoffdruck hydriert. Es wird filtriert, eingedampft und destilliert, worauf man in einer Ausbeute von 94,5 % d.Th. 1-Amino-2,3-propandiol erhält.

Beispiel 4

Ein Gemisch aus 170 kg Dihydroxyaceton, 138 kg Ethanolamin und 1400 l Methanol wird zwei Stunden bei Raumtemperatur gerührt.

Anschließend fügt man dem Reaktionsgemisch 150 kg Raney-Nickel zu und hydriert während 2 Stunden unter 100 bar Wasserstoffdruck bei 40° bis zum Stillstand der Wasserstoffaufnahme.

Man filtriert den Katalysator ab, dampft unter vermindertem Druck ein und unterwirft den Rückstand der Destillation. Bei 152-157°/0,001 mm geht 2-[(2-Hydroxyethyl)amino]-1,3-propandiol über (224 kg; 88 % d.Th.).

**Patentansprüche**

1.  Verfahren zur Herstellung von Hydroxyaminen der Formel I,

    $R^1$-CH(OH)-CH($NHR^3$)-$R^2$    I

    worin $R^1$ und $R^2$ jeweils unabhängig voneinander H, $CH_2OH$ und $R^3$ H, $C_1$-$C_4$ Alkyl, $C_1$-$C_4$ Hydroxyalkyl bedeuten, dadurch gekennzeichnet, daß man eine Oxoverbindung der Formel II,

    $R^1$-CH(OH)-CO-$R^2$    II

    worin $R^1$, $R^2$ die für Formel I angegebene Bedeutung besitzen, mit einem Amin $R^3$-$NH_2$, worin $R^3$ die für Formel I angegebene Bedeutung hat, zu einem Ketimin der Formel III umsetzt,

    $R^1$-CH(OH)-C(=$NR^3$)-$R^2$    III

    worin $R^1$, $R^2$ und $R^3$ die für Formel I angegebene Bedeutung besitzen, und dieses anschließend mit einem Reduktionsmittel behandelt.

2.  Verfahren zur Herstellung von 2-Amino-1,3-propandiol nach Anspruch 1, dadurch gekennzeichnet, daß man 1,3-Dihydroxyaceton mit Ammoniak zu 2-Imino-1,3-propandiol umsetzt und anschließend mit einem Reduktionsmittel behandelt.

3. Verfahren nach Anspruch 1, dadurch gekennzeichnet, daß man als Reduktionsmittel Wasserstoff in Gegenwart eines Metallkatalysators verwendet.

**Claims**

1. Process for the preparation of hydroxyamines of the formula I

   $$R^1\text{-}CH(OH)\text{-}CH(NHR^3)\text{-}R^2 \qquad I$$

   wherein $R^1$ and $R^2$ independently of one another are each H or $CH_2OH$ and $R^3$ is H, $C_1$-$C_4$-alkyl or $C_1$-$C_4$-hydroxyalkyl, characterised in that an oxo compound of the formula II

   $$R^1\text{-}CH(OH)\text{-}CO\text{-}R^2 \qquad II$$

   wherein $R^1$ and $R^2$ have the meaning indicated for formula I is reacted with an amine $R^3$-$NH_2$ wherein $R^3$ has the meaning indicated for formula I to give a ketimine of the formula III

   $$R^1\text{-}CH(OH)\text{-}C(=NR^3)\text{-}R^2 \qquad III$$

   wherein $R^1$, $R^2$ and $R^3$ have the meaning indicated for formula I, and this ketimine is then treated with a reducing agent.

2. Process for the preparation of 2-amino-1,3-propanediol according to Claim 1, characterised in that 1,3-dihydroxyacetone is reacted with ammonia to give 2-imino-1,3-propanediol and the latter is then treated with a reducing agent.

3. Process according to Claim 1, characterised in that the reducing agent used is hydrogen in the presence of a metal catalyst.

**Revendications**

1. Procédé de préparation d'hydroxylamines de formule I,

   $$R^1\text{-}CH(OH)\text{-}CH(NHR^3)\text{-}R^2 \qquad I$$

   dans laquelle $R^1$ et $R^2$ désignent respectivement, indépendamment l'un de l'autre, H, $CH_2OH$ et $R^3$ désigne H, un radical alkyle $C_1$-$C_4$ ou hydroxyalkyle $C_1$-$C_4$, caractérisé en ce qu'un oxocomposé de formule II,

   $$R^1\text{-}CH(OH)\text{-}CO\text{-}R^2 \qquad II$$

   dans laquelle $R^1$ et $R^2$ possèdent la signification mentionnée pour la formule I est amené à réagir avec une amine $R^3$-$NH_2$, dans laquelle $R^3$ a la signification mentionnée pour la formule I, pour former une cétimine de formule III,

$$R^1\text{-}CH(OH)\text{-}C(=NR^3)\text{-}R^2 \qquad III$$

dans laquelle $R^1$, $R^2$ et $R^3$ ont la signification mentionnée à la formule I qui est ensuite traitée avec un réducteur.

2. Procédé de préparation de 2-amino-1,3-propanediol selon la revendication 1, caractérisé en ce de la 1,3-dihydroxyacétone est amenée à réagir avec de l'ammoniac pour former du 2-imino-1,3-propanediol qui est ensuite traité avec un réducteur.

3. Procédé selon la revendication 1 caractérisé en ce que le réducteur utilisé est de l'hydrogène en présence d'un catalyseur métallique.